# FASCICULE DE BREVET EUROPEEN

(11) **EP 2 103 261 B1**
(45) Date de publication et mention de la délivrance du brevet: **05.01.2011**
(21) Numéro de dépôt: 09155739.7
(22) Date de dépôt: 20.03.2009
(51) Int. Cl.: A61B 17/04

(54) **Dispositif implantable**
Implantat
Implantable device

(30) Priorité: 21.03.2008 FR 0851862
(43) Date de publication de la demande: 23.09.2009
(73) Titulaire: Cousin Biotech, 59117 Wervicq Sud (FR)
(72) Inventeur: Solecki, Gilles, 59390 Lannoy (FR); Dalle, Valery, 59170, Croix (FR); Leroy, Joel, 62172 Bouvigny Boyeffles (FR); Dallemagne, Bernard, 67000 Strasbourg (FR); Marescaux, Jacques, 67310 Scharrachbergheim (FR)
(74) Mandataire: Cochonneau, Olivier

(56) Documents cités:
- WO-A-2005/016176
- WO-A-2007/002071
- WO-A-2007/067206
- US-A- 4 326 531
- US-A- 5 370 661
- US-A1- 2007 049 971

## Description

La présente invention concerne le domaine technique des dispositifs implantables pour le rapprochement de structures anatomiques fragiles, tels que les piliers du diaphragme dans le traitement de la hernie hiatale.

A l'état normal, l'orifice hiatal est traversé par le segment distal de l'oesophage accompagné des nerfs vague antérieur et postérieur. La jonction entre l'estomac et l'oesophage (cardia) est normalement située en dessous du diaphragme. Le muscle diaphragmatique sépare la cavité abdominale et la cavité thoracique. Il présente trois orifices qui permettent le passage de la veine cave, de l'aorte et de l'oesophage. L'orifice de passage de l'oesophage est dénommé orifice hiatal. Il est formé essentiellement par le piler diaphragmatique droit, avec une contribution plus faible du pilier gauche. Le pilier droit prend son origine au niveau du ligament vertébral longitudinal antérieur qui couvre les vertèbres lombaires.

Une hernie hiatale est provoquée par une altération de l'anatomie de l'orifice hiatal. Des structures anatomiques normalement confinées dans la cavité abdominale passent alors au travers dudit orifice déformé et se retrouvent en position intra-thoracique.

Quatre types de hernie hiatale peuvent alors survenir, répertoriées selon leur gravité croissante :
- le premier type est une hernie hiatale par glissement. L'orifice hiatal est élargi et la membrane phréno-oesophagienne distendue ce qui permet l'ascension de la jonction gastro-oesophagienne au dessus du diaphragme. La plupart des hernies de ce type sont asymptomatiques. Elles peuvent cependant entraîner l'apparition d'une maladie de reflux gastro-oesophagien par la perte de la compétence du mécanisme sphinctérien oesophagien inférieur.
- le second type est une hernie para-oesophagienne. La jonction gastro-oesophagienne demeure en place, mais la partie supérieure de l'estomac (fundus) migre vers le thorax au travers d'une zone de faiblesse de la membrane phréno-oesophagienne. Ce type de hernie peut entraîner des complications mécaniques ou vasculaires au niveau du bas oesophage ou de l'estomac hernié ;

- le troisième type est une hernie mixte. Elle combine des éléments des deux premiers types. Dans certains cas, la totalité de l'estomac peut se retrouver en position sus-diaphragmatique ;
- le quatrième type est caractérisé par un élargissement important de l'orifice diaphragmatique, permettant la herniation, non seulement de l'estomac, mais également d'autres structures abdominales (colon, rate).

Le traitement d'une hernie hiatale, quel que soit le type de hernie, comporte la réduction de la hernie, c'est-à-dire le repositionnement des viscères herniés en dessous du diaphragme, et la plastie de l'orifice hiatal élargi.

La plastie de l'orifice hiatal consiste en la recalibration de l'orifice hiatal autour de l'oesophage, sans comprimer mécaniquement l'oesophage tout en assurant une fermeture satisfaisante qui doit empêcher une récidive de la hernie. Cette réparation est appliquée sur une structure musculaire (piliers du diaphragme) qui n'a pas la résistance d'une structure aponévrotique et qui est sollicitée par des variations de pressions intra-abdominales (positives) et thoraciques (négatives).

La localisation de l'orifice hiatal (postérieure) accroît la difficulté du geste technique.

Une première technique de plastie des hernies hiatales consiste à suturer avec des fils de suture non résorbables les piliers du diaphragme, en avant et/ou en arrière de l'oesophage. Des languettes, généralement en PTFE, peuvent être intercalées entre le fil de suture et les piliers du diaphragme pour éviter de cisailler les structures musculaires, cette technique de suture est alors appelée suture appuyée. Malgré cette précaution, on observe un effet de cisaillement des piliers gauche et droit, particulièrement avec la technique de suture simple. Cette première technique réalisée par laparoscopie rapporte des taux de récidive jusqu'à 42 % supérieurs aux taux de récidive observés dans la chirurgie conventionnelle, c'est-à-dire par laparotomie. Il n'existe pas d'étude randomisée comparant la suture simple et la suture appuyée. La différence des taux de récidive entre la chirurgie ouverte et la chirurgie laparoscopique laisse suspecter l'existence de difficultés techniques propres à la chirurgie laparoscopique.

Une seconde technique consiste à renforcer la suture des piliers en la recouvrant d'une prothèse qui entoure partiellement ou totalement l'oesophage. Pour être efficace, la prothèse est invariablement proche de la paroi de l'oesophage. Une étude réalisée sur une durée courte -6 mois- démontre un taux de récidive de 9% après renforcement au moyen d'une prothèse contre 24% pour la suture simple. Ces résultats favorables doivent être contre-balancés par la description de complications sévères liées à l'utilisation du matériel prothétique. Un contact direct entre la prothèse et la paroi de l'oesophage peut provoquer soit une réaction inflammatoire fibreuse qui entraîne une sténose de l'oesophage, soit une érosion progressive de la paroi oesophagienne avec pénétration de la prothèse dans la lumière de l'oesophage. Ces complications nécessitent une reprise chirurgicale pouvant aller jusqu'à la résection de l'oesophage. La probabilité d'observer ces complications est majorée par le fait que le contact prothèse-oesophage est dynamique, entre les mouvements respiratoires du diaphragme et les mouvements péristaltiques de l'oesophage, non synchronisés.

Une troisième technique consiste à disposer une prothèse sur l'orifice hiatal sans tension, c'est-à-dire sans suture préalable des piliers. Aucun suivi à long terme n'a été rapporté à ce jour sur cette dernière technique.

FR 2.889.441 A1 décrit une prothèse de renfort utilisée pour la mise en oeuvre des seconde et troisième techniques. Cette prothèse a une forme de collerette au centre de laquelle est disposé l'oesophage.

Le preambule de la revendication 1 est basé sur le document WO 2005/016176.

De nombreuses études ont démontré que la récidive de la hernie hiatale est la cause principale d'échec du traitement chirurgical du reflux gastro-oesophagien et de la hernie hiatale. Elle est à l'origine de la majorité des reprises chirurgicales. La récidive est provoquée par la rupture, la distension ou une malfaçon de la plastie crurale.

Trois facteurs doivent être pris en considération lors du traitement d'une hernie hiatale :
- la longueur de l'oesophage. Elle doit être suffisante pour permettre le repositionnement sans tension de la jonction gastro-oesophagienne sous le diaphragme ;
- la qualité des piliers du diaphragme. En cas de hernie volumineuse, les piliers peuvent être très écartés et réduits à deux fines bandes musculaires fragiles ;
- la qualité de la plastie de l'orifice hiatal. 90 à 95% des hernies observées dans la maladie de reflux gastro-oesophagien sont des hernies du premier type décrit ci-dessus et donc de petite taille, avec conservation d'une musculature satisfaisante. La situation anatomique de l'orifice hiatal peut rendre difficile un geste de suture laparoscopique dont la qualité dépend significativement de l'expérience de l'opérateur.

La présente invention a pour objet un dispositif implantable palliant les problèmes précités pour le rapprochement de structures anatomiques fragiles, notamment de structures musculaires telles que la paroi abdominale , les piliers du diaphragme dans le traitement de la hernie hiatale, le coeur, l'estomac ou l'utérus, ou de structures parenchymateuses telles que les reins, le foie et les poumons, comprenant au moins un premier élément longiligne de rapprochement d'au moins deux desdites structures anatomiques comportant sur sa longueur une première pièce d'appui, fixe ou fixable dans une position donnée dudit premier élément, au moins une pièce de blocage fixée à une distance donnée D de ladite première pièce d'appui dans ladite position, et une seconde pièce d'appui qui est apte à coulisser sur la longueur dudit élément longiligne en sorte que, lors de l'implantation, ladite pièce de blocage est forcée à travers ladite seconde pièce d'appui et vient en butée contre ladite seconde pièce d'appui, les première et seconde pièces d'appui délimitant entre elles un espace ayant au moins une dimension D' inférieure ou égale à D et apte à recevoir et enserrer lesdites structures anatomiques. La seconde pièce d'appui présente un premier orifice permettant le passage du premier élément longiligne et comporte une première pièce tronconique creuse ayant des découpes formant des ailettes latérales et ayant un évidement central qui prolonge ledit premier orifice de passage, le diamètre de sortie de l'évidement de la première pièce tronconique étant inférieur à l'encombrement de ladite pièce de blocage en sorte que le passage à force de ladite pièce de blocage à travers la seconde pièce d'appui est obtenu grâce à la déformation radiale des ailettes. La première pièce tronconique est pourvue d'un protecteur ayant une largeur de l'ordre de la hauteur de ladite pièce tronconique, éventuellement majorée de tout ou partie de la hauteur de ladite pièce de blocage, et apte à être disposé en fonctionnement sur la première pièce tronconique, et éventuellement ladite pièce de blocage, en sorte de recouvrir au moins lesdites découpes latérales.

On entend par structure anatomique fragile, toute structure anatomique à l'exception des os, et en particulier les structures musculaires et parenchymateuses.

Le premier orifice permet à la seconde pièce d'appui de coulisser librement sur la longueur dudit premier élément longiligne.

La première pièce tronconique s'étend de préférence de la face extérieure de ladite seconde pièce d'appui de sorte que celle-ci conserve une face intérieure sensiblement plane pour ne pas cisailler les structures anatomiques.

Le protecteur évite que le premier élément longiligne, et éventuellement la pièce de blocage, ne passent à travers l'une des découpes latérales lors du passage en force de ladite pièce de blocage occasionnant la déformation desdites ailettes ou une fois le dispositif implanté, et ne viennent en contact avec la surface extérieure des structures anatomiques afin d'éviter tout risque de cisaillement et gêner la manipulation du dispositif par le praticien.

La pièce de blocage, en particulier lorsqu'elle comporte une extrémité amincie voire pointue, est de préférence recouverte par ledit protecteur pour éviter de perforer des structures anatomiques voisines.

S'agissant des piliers du diaphragme qui sont des structures anatomiques très tendres et fragiles, en particulier parce que les fibres des muscles sont toutes orientées dans la direction longitudinale des piliers, compte tenu de la zone d'implantation soumise aux mouvements du diaphragme (environ 2500 pulsations du diaphragme par jour), la demanderesse a observé que, sans protecteur, si le premier élément longiligne et éventuellement la pièce de blocage passent à travers une découpe latérale et viennent en contact avec le pilier recevant la seconde pièce d'appui, ce pilier est cisaillé transversalement par ces derniers et le rapprochement des piliers opéré grâce au dispositif est alors nul. Le protecteur empêche ainsi tout cisaillement des piliers du diaphragme et permet que le rapprochement des piliers opéré par le dispositif selon l'invention soit parfaitement stable.

Contrairement aux techniques de sutures simples et appuyées encerclant en partie au moins deux structures anatomiques, telles que les piliers gauche et droit, l'élément longiligne traverse lesdites structures anatomiques, lesquelles sont maintenues entre les première et seconde pièces d'appui. Il n'y a pas de fil de suture venant en appui sur la surface extérieure desdites structures anatomiques, ce qui élimine les risques de cisailler ces derniers qui sont des structures fragiles, particulièrement en ce qui concerne les piliers du diaphragme qui sont des muscles « tendres ». Lesdites structures anatomiques, par exemple les piliers, sont enserrées entre les pièces d'appui bloquées et distantes selon une distance déterminée D' inférieure ou égale à D de sorte que le praticien connaît précisément la distance selon laquelle il a rapproché lesdites structures anatomiques augmentant ainsi la précision de la technique opératoire.

Dans le cas du traitement de la hernie hiatale, le dispositif une fois implanté ne comporte pas de bords extérieurs susceptibles d'entrer en contact avec la paroi oesophagienne. Le dispositif est facile à mettre en place contrairement aux prothèses et sutures utilisés dans l'état de la technique ce qui diminue les risques post-opératoires de récidive. De plus, ledit dispositif étant peu encombrant peut être implanté facilement par laparoscopie. Les piliers gauche et droit viennent en appui contre les faces intérieures des première et seconde pièces d'appui et sont retenus dans leur position resserrée entre ces dernières. Il est bien sûr possible d'implanter le dispositif selon la présente invention par laparotomie.

La première pièce d'appui et la ou les pièces de blocage sont fixées ou fixables sur la longueur dudit élément longiligne par n'importe quels moyens mécaniques ou chimiques connus de l'état de la technique : clipsage, collage, soudures ultrasons ou haute fréquences..., ou encore en réalisant un noeud sur lui-même dudit élément longiligne faisant office de pièce de blocage ou de butée arrière pour ladite première pièce d'appui.

Dans une variante, les première et seconde pièces d'appui ont au moins une face intérieure sensiblement plane, de préférence ladite face intérieure a une forme générale d'ellipse.

Les faces intérieures planes des première et seconde pièces d'appui sont plaquées contre les structures anatomiques, de sorte qu'elles ne peuvent cisailler la structure musculaire fibreuse de ces dernières lorsqu'il s'agit de muscles, en particulier des piliers du diaphragme.

De préférence, la première pièce d'appui a une forme générale sensiblement plane.

Dans une variante, le dispositif comprend un second élément longiligne de rapprochement desdites structures anatomiques, la première pièce d'appui étant fixe ou fixable dans une position donnée dudit second élément, et au moins une pièce de blocage fixée sur la longueur dudit second élément à une distance donnée D de ladite première pièce d'appui dans ladite position, la seconde pièce d'appui étant apte à coulisser sur la longueur dudit second élément longiligne en sorte que, lors de l'implantation, ladite pièce de blocage est forcée à travers ladite seconde pièce d'appui et vient en butée contre ladite seconde pièce d'appui.

La seconde pièce d'appui est apte à coulisser à la fois sur le premier élément longiligne et sur le second élément longiligne.

L'emploi d'un second élément longiligne permet de renforcer la pression exercée de part et d'autre desdites structures anatomiques respectivement par les première et seconde pièces d'appui.

Le second élément traverse également lesdites structures anatomiques de sorte qu'il ne peut cisailler leurs surfaces extérieures.

Lorsque les structures anatomiques sont les deux piliers du diaphragme, le choix du nombre d'élément longiligne est fonction du renfort à apporter à la plastie du hiatus oesophagien et du type de hernie hiatale.

Dans une variante, le premier, et éventuellement le second, élément longiligne comporte plusieurs pièces de blocage successives, de préférence ayant une hauteur du même ordre, à des distances prédéterminées D de ladite première pièce d'appui, de préférence la distance D est comprise dans l'intervalle [10 ; 25] mm.

Le premier, et éventuellement le second, élément longiligne comporte de préférence quatre pièces de blocage, la première étant à une distance D de préférence de l'ordre de 10 mm de la première pièce d'appui, les pièces de blocage suivantes étant espacées les unes des autres de préférence de l'ordre de 5 mm à compter de ladite première pièce de blocage. Les pièces de blocage sont autant de repère pour le praticien pour bloquer la seconde pièce d'appui et déterminer précisément la distance D' selon laquelle les structures anatomiques sont rapprochées ce qui n'était pas possible avec les sutures et orthèses mis en oeuvre dans l'état de la technique, particulièrement pour le traitement de la hernie hiatale.

Les pièces de blocage disposées sur les premier et second éléments longilignes sont de préférence à égales distances de ladite première pièce d'appui de sorte que le praticien force deux pièces de blocage à la fois à travers la seconde pièce d'appui en sorte de former un espace délimité entre les première et seconde pièces d'appui ayant au moins une dimension D' sensiblement constante.

La hauteur d'une pièce de blocage correspond à la longueur d'une portion de premier, ou de second, élément longiligne selon laquelle ladite pièce est fixée. De préférence, les pièces de blocage sont identiques et présentent une hauteur du même ordre.

Dans une variante, la seconde pièce d'appui présente un second orifice permettant le passage dudit second élément longiligne.

Le second orifice permet à la seconde pièce d'appui de coulisser librement sur la longueur dudit second élément longiligne.

Dans une variante, la seconde pièce d'appui comporte une seconde pièce tronconique creuse, ayant des découpes formant des ailettes latérales et ayant un évidement central qui prolonge le second orifice de passage. Le diamètre de sortie de l'évidement de ladite seconde pièce tronconique est inférieur à l'encombrement de la pièce de blocage, en sorte que le passage en force de la pièce de blocage à travers la seconde pièce d'appui est obtenu grâce à la déformation radiale des ailettes.

La seconde pièce tronconique creuse s'étend de préférence de la face extérieure de ladite seconde pièce d'appui de sorte que celle-ci conserve une face intérieure sensiblement plane pour ne pas cisailler les structures anatomiques.

Dans une variante, la première, et éventuellement la seconde, pièce tronconique creuse comporte un épaulement interne dans sa portion d'extrémité formant un logement apte à recevoir tout ou partie de la hauteur d'une pièce de blocage.

Selon la profondeur de l'épaulement interne, tout ou partie de la hauteur de la pièce de blocage est logée à l'intérieur de la première, ou de la seconde, pièce tronconique et ce dans l'évidement central.

Les découpes latérales sont ainsi en partie, ou totalement selon la profondeur de l'épaulement, obturées par la pièce de blocage en sorte que l'élément longiligne ne puisse passer à travers lesdites ailettes et cisailler des structures anatomiques environnantes.

Dans une variante, ladite seconde pièce tronconique est pourvue d'un protecteur ayant une largeur de l'ordre de la hauteur de ladite seconde pièce tronconique, éventuellement majorée de tout ou partie de la hauteur de l'une des pièces de blocage, et apte à être disposée en fonctionnement sur ladite seconde pièce tronconique et éventuellement ladite pièce de blocage, en sorte de recouvrir au moins lesdites découpes latérales.

Lorsque le dispositif comprend deux éléments longilignes, la seconde pièce d'appui comprend de préférence deux pièces tronconiques telles que décrites ci-dessus et deux desdits protecteurs, chacun étant apte à être disposé en fonctionnement sur les pièces tronconiques.

De préférence, la largeur du protecteur est déterminée en sorte qu'il recouvre à la fois les découpes latérales et tout ou partie de la hauteur de la pièce de blocage sélectionnée. Lorsque la première, et éventuellement la seconde, pièce tronconique comporte un épaulement interne, la largeur du protecteur correspond à la hauteur de ladite pièce tronconique majorée de la hauteur de la pièce de blocage moins la profondeur dudit épaulement. Cette disposition permet que la pièce de blocage, en particulier lorsqu'elle comporte une extrémité amincie voire pointue, soit recouverte par ledit protecteur pour éviter de perforer des structures anatomiques voisines.

Ce protecteur évite que le second élément longiligne, et éventuellement la pièce de blocage, ne passent à travers l'une des découpes latérales et ne viennent en contact avec la surface extérieure des structures anatomiques afin d'éviter tout risque de cisaillement.

Dans une variante, le protecteur de la première pièce tronconique creuse, et éventuellement le protecteur de la seconde pièce tronconique creuse, consiste dans une bande dans un matériau élastique, de préférence à base de silicone.

Le protecteur peut être maintenu sur la première pièce tronconique simplement par sa force élastique ou collé, de préférence selon sa base sur la face extérieure de la seconde pièce d'appui correspondant à sa face non orientée en fonctionnement vers les structures anatomiques à rapprocher.

Le protecteur, bien que plaqué contre la pièce tronconique en sorte de recouvrir au moins lesdites découpes latérales de cette dernière, est suffisamment élastique afin de permettre aux ailettes latérales de se déformer radialement lors du passage en force de la pièce de blocage à travers l'orifice de passage.

Le protecteur peut ainsi être sélectionné indépendamment des autres pièces du dispositif (première et seconde pièces d'appui, première et seconde pièces tronconiques), notamment de par sa largeur, en fonction de la hauteur de la pièce tronconique et/ou de la hauteur de la pièce de blocage.

Dans une variante, le premier, et éventuellement le second, élément longiligne est un monofilament, une tresse ou un câble rigide de faible diamètre, de préférence de l'ordre de 1,7 mm.

Dans une variante, le premier, et éventuellement le second, élément longiligne présente une extrémité rigide et courbe permettant son introduction dans lesdites structures anatomiques, notamment à l'opposé de ladite première pièce d'appui.

La rigidité doit être suffisante pour que ladite extrémité puisse traverser lesdites structures anatomiques et notamment les piliers. Ladite extrémité peut être formée à partir du premier ou du second élément longiligne lui-même par enduction ou imprégnation d'un polymère implantable puis mise en forme, ou par fusion du ou des polymères formant le dit premier ou second élément à l'aide d'un solvant de ces derniers. Ladite extrémité peut être également formée en rapportant sur l'extrémité libre une pièce courbe et rigide implantable moulée.

Dans une variante, la ou les pièces de blocage ont une forme générale tronconique.

Dans une variante, les première et seconde pièces d'appui, la ou les pièces de blocage, la première pièce tronconique creuse, éventuellement la seconde pièce tronconique creuse, le protecteur de la première tronconique creuse, éventuellement le protecteur de la seconde pièce tronconique creuse, sont moulés, de préférence dans un polymère choisi parmi les polymères suivants : PEEK (polyétheréthercetone), POM (polyoxyméthylène), PET (polyéthylènetéréphtalate), PP (polypropylène), PE (polyéthylène).

Ces différentes pièces sont moulées en même temps en sorte de former une pièce unique. Le protecteur, étant dans ce cas rigide, se présente sous la forme d'un cylindre s'étendant de la face de la seconde pièce d'appui non orientée en fonctionnement vers les structures anatomiques à rapprocher. De préférence, le protecteur recouvre au moins les découpes latérales de la pièce tronconique creuse sans entrer en contact direct avec ladite pièce tronconique et donc ses ailettes de sorte de ménager un espace libre, délimité entre ladite pièce tronconique et ledit protecteur, dans lequel lesdites ailettes peuvent se déformer radialement lors du passage en force de la pièce de blocage.

L'invention concerne selon un deuxième aspect un ensemble pour le rapprochement de structures anatomiques fragiles, notamment de structures musculaires telles que la paroi abdominale, les piliers du diaphragme dans le traitement de la hernie hiatale, le coeur, l'estomac ou l'utérus ou de structures parenchymateuses telles que les reins, le foie et les poumons comportant un dispositif implantable selon l'une des variantes de réalisation décrites ci-dessus et un kit d'implantation comprenant notamment un organe de traction et un poussoir.

L'invention concerne selon un troisième aspect, l'utilisation d'un ou plusieurs dispositifs implantables selon l'une des variantes de réalisation décrites ci-dessus, pour le rapprochement de structures musculaires telles que la paroi abdominale, le diaphragme, le coeur, l'estomac ou l'utérus.

L'invention concerne selon un quatrième aspect, l'utilisation d'un ou plusieurs dispositifs implantables selon l'une des variantes de réalisation décrites ci-dessus, pour le rapprochement de structures parenchymateuses telles que les reins, le foie et les poumons.

L'invention sera mieux comprise, grâce à la description ci-après, qui se rapporte à deux modes de réalisation préférés, donné à titre d'exemples non limitatifs, et expliqués avec références aux dessins schématiques annexés, dans lesquels :
- la figure 1A est une représentation schématique en perspective et vue de côté d'un premier exemple de dispositif selon la présente invention ;
- la figure 1B est une représentation schématique en perspective et vue de côté de la seconde pièce d'appui représentée à la figure 1A ;
- la figure 1C est une représentation schématique vue selon la face intérieure de la seconde pièce d'appui représentée à la figure 1B ;
- la figure 1D est une représentation schématique vue de coté du protecteur de la pièce tronconique de la seconde pièce d'appui représentée aux figures 1B et 1C ;
- la figure 2 est une représentation schématique d'une hernie hiatale du second type ;
- les figures 3 à 8 sont des représentations schématiques des différentes étapes pour l'implantation du dispositif décrit aux figures 1A, 1B et 2 ;
- les figures 9 et 10 sont des représentations schématiques en perspective d'une variante de la pièce tronconique représentée aux figures 1B-1D ;
- les figures 11 et 12 sont des représentations schématiques vues de côté d'un second mode de réalisation du dispositif selon la présente invention.
- la figuré 13 est une représentation schématique en perspective d'une variante du protecteur représenté à la figure 10.

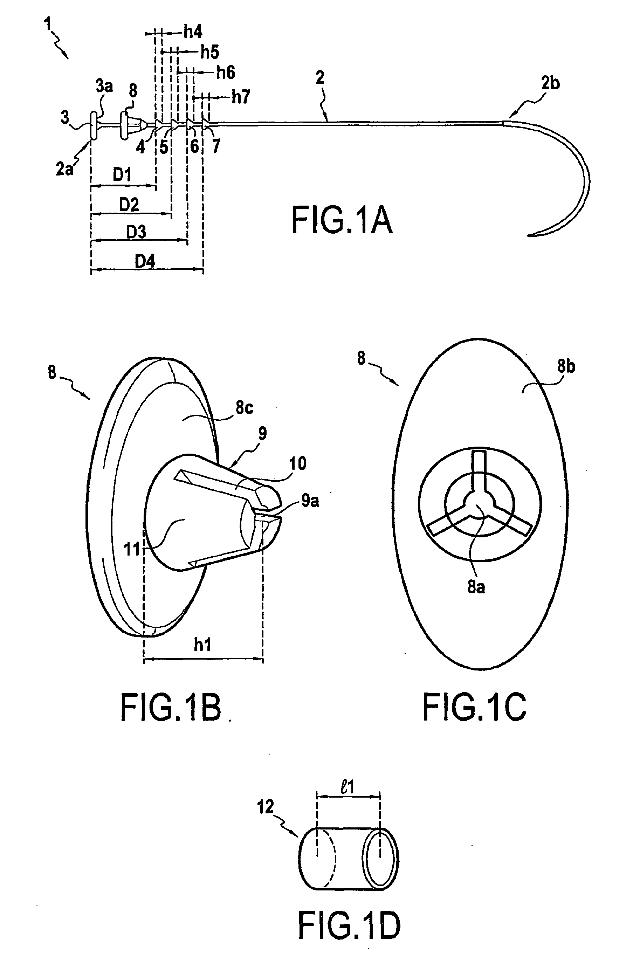

Le dispositif implantable 1 représenté à la figure 1A est destiné au traitement de la hernie hiatale. Il comprend un élément longiligne de rapprochement 2, lequel comprend sur sa longueur une première pièce d'appui 3, fixe et terminant l'une des extrémités 2a dudit élément longiligne 2, et quatre pièces de blocage 4,5,6,7 successives. Les première 4, seconde 5, troisième 6 et quatrième 7 pièces de blocage sont respectivement à des distances D1,D2,D3,D4 de ladite première pièce d'appui 3. Dans cet exemple particulier, D1,D2,D3,D4 valent respectivement 10 mm, 15 mm, 20 mm et 25 mm. Les pièces de blocage 4,5,6,7 ont respectivement une hauteur h4,h5,h6,h7 du même ordre, dans cet exemple précis de l'ordre de 5 mm. Les pièces de blocage 4,5,6,7 ont une forme générale tronconique dont la plus petite base est orientée sur ledit élément longiligne 2 dans une direction opposée à la première pièce d'appui 3. La première pièce d'appui 3 et les pièces de blocage 4,5,6,7 sont fixées sur la longueur dudit élément longiligne 2 par n'importe quels moyens connus de l'état de la technique, par exemple par collage.

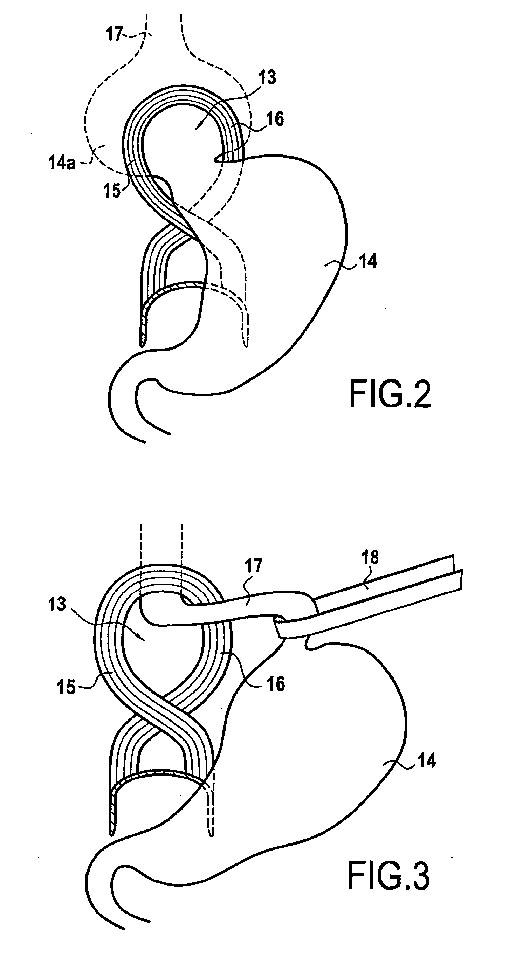

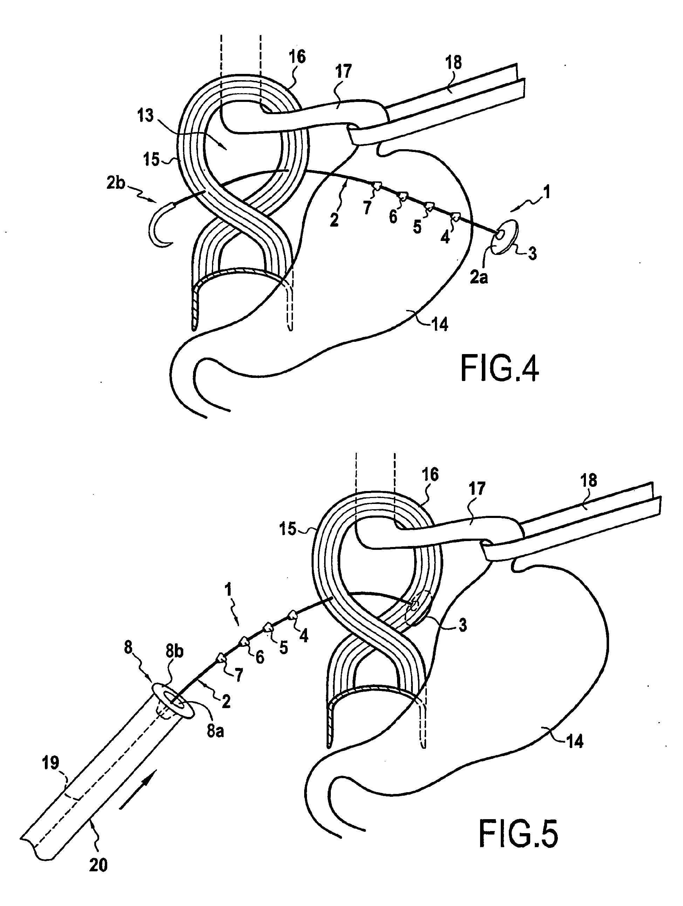

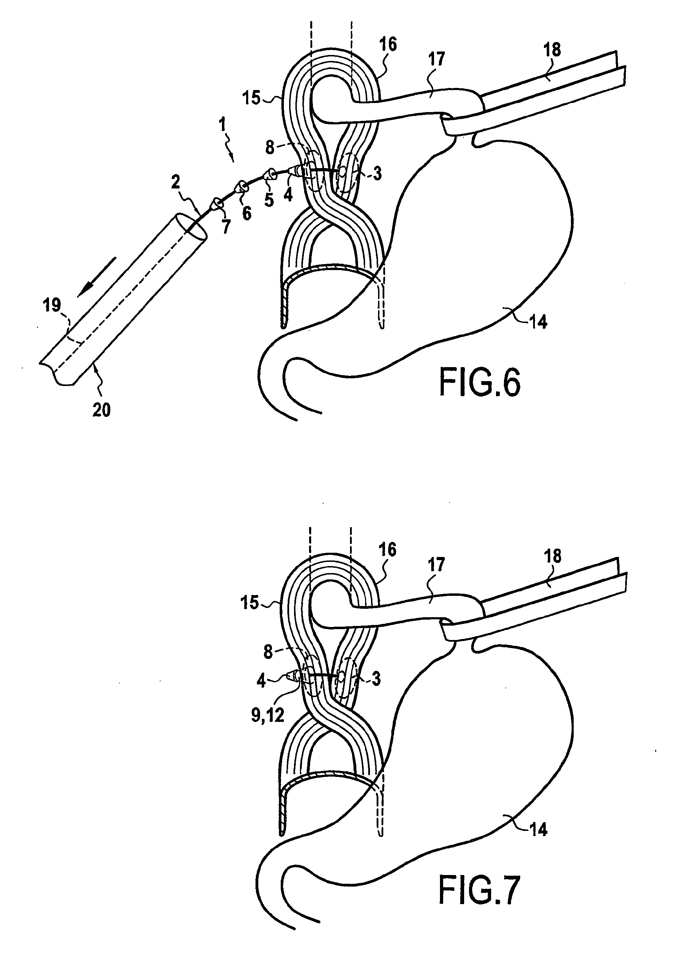

Le dispositif 1 comprend une seconde pièce d'appui 8, représentée à la figure 1B, ayant un orifice 8a permettant le passage dudit élément longiligne 2. Les première 3 et seconde 8 pièces d'appui ont leurs faces intérieures 3a,8b sensiblement planes et ont, vue selon leurs face intérieures 3a,8b, une forme générale d'ellipse. La première pièce d'appui 3 a une forme générale plane. La seconde pièce d'appui 8 comporte une pièce tronconique creuse 9 s'étendant de sa face extérieure 8c. La pièce tronconique 9 comporte des découpes 10 formant des ailettes latérales 11 et ayant un évidement central 9a qui prolonge l'orifice de passage 8a. Le diamètre de sortie de l'évidement 9a de ladite pièce tronconique 9 est inférieur à l'encombrement des pièces de blocage 4,5,6,7. L'élément longiligne 2 présente une extrémité rigide et courbe 2b à l'opposé de ladite première pièce d'appui 3. Le dispositif 1 comprend également un protecteur se présentant sous la forme d'une bande 12 dans un matériau élastique et implantable, de préférence à base de silicone, ayant une largeur ℓ1 de l'ordre de la hauteur h1 de ladite pièce tronconique 9, de préférence de l'ordre de la hauteur h1 ajoutée à la hauteur h4,h5,h6,h7 de l'une des pièces de blocage 4,5,6,7. Ladite bande 12 est disposée en fonctionnement sur la pièce tronconique 9 en sorte de recouvrir non seulement les découpes latérales 10, mais également la pièce de blocage 4,5,6,7 afin de protéger les structures anatomiques proches d'éventuelles perforations dues à l'extrémité amincie, globalement en forme de pointe, de la pièce de blocage 4,5,6,7. La figure 2 est une représentation schématique d'une hernie hiatale 13 mettant en évidence la remontée par le hiatus oesophagien 13 d'une partie 14a de l'estomac 14 suite à une distension entre les piliers 15,16 du diaphragme. Le dispositif 1 est implanté de préférence par laparoscopie. En premier, la base de l'oesophage 17 est écartée au moyen d'un lac 18 maintenu sous tension et se présentant sous la forme d'un cordon pour dégager les piliers 15,16. Puis l'élément longiligne 2 est introduit à travers les piliers 15,16, sensiblement en leurs centres, à partir de son extrémité courbe 2b faisant office de moyen d'introduction dudit élément longiligne 2 tel que représenté à la figure 4. L'élément longiligne 2 est introduit ainsi jusqu'à ce que la première pièce d'appui 3 vienne en appui et en butée contre le pilier 16. La face intérieure plane 3a de la première pièce d'appui 3 vient contact avec la surface extérieure dudit pilier 16 tel que représenté à la figure 5. Puis le praticien met en tension ledit élément longiligne 2 au moyen d'un organe de traction 19 passé par le conduit intérieur d'un poussoir 20 et attaché à son extrémité courbe 2b. La seconde pièce d'appui 8 peut être enfilée facilement à partir de l'extrémité courbe et rigide 2b puis poussée au travers dudit conduit intérieur au moyen d'un piston actionné par le praticien. Le praticien dispose alors la seconde pièce d'appui 8 contre le second pilier 15 en actionnant à la fois l'organe de traction 19 mettant en tension ledit élément longiligne 2 et le piston pour passer en force la seconde pièce 8 sur chaque pièce de blocage jusqu'à la pièce de blocage prédéterminée par le praticien selon le degré de distension des piliers 15,16. La seconde pièce d'appui 8 est introduite de sorte que sa face intérieure plane 8b vienne en contact avec le second pilier 15. Lors du passage en force de chacune des pièces de blocage à travers la seconde pièce d'appui 8, les ailettes 11 de la pièce tronconique 9 se déforment radialement pour son passage, dans l'exemple illustré la quatrième pièce de blocage 4 vient en butée contre la seconde pièce d'appui 8. Selon le degré de distension entre les piliers 15,16, le praticien ajuste le rapprochement des deux piliers 15,16 aux moyens des pièces de blocage qu'il force une à une à travers ladite seconde pièce d'appui 8. Sur la figure 6, la seconde pièce 8 a été passée en force par-dessus les quatre pièces de blocage 4,5,6,7. L'espace délimité entre lesdites pièces d'appui 3,8 présente ainsi au moins une dimension D' inférieure à 10 mm apte à recevoir et enserrer les deux piliers 15,16 du diaphragme. Puis le praticien coupe l'élément longiligne 2 au ras de la quatrième pièce de blocage 4 venant en butée contre la seconde pièce d'appui 8 et retire la portion d'élément longiligne 2 coupée ainsi que le poussoir 20 et le lac 18. De préférence, la bande 12 est pré-positionnée sur la pièce tronconique 9 avant l'implantation. Ainsi, à ce stade, la bande élastique 12 empêche lors du passage en force des pièces de blocage 4,5,6,7, occasionnant la déformation des ailettes, que l'élément longiligne 2 ne passe à travers l'un des découpes latérales et ne vienne cisailler les piliers 15,16 et/ou gêner la manipulation du dispositif 1 par le praticien.

Le dispositif 1 ainsi implanté est parfaitement maintenu en tension par la dite pièce de blocage 4, laquelle maintient également les faces intérieures planes 3a,8b contre les piliers fibreux 15,16. Le dispositif 1 ne comporte pas de partie susceptible de cisailler la surface extérieure des piliers 15,16. Les piliers 15,16 sont maintenus rapprochés durablement.

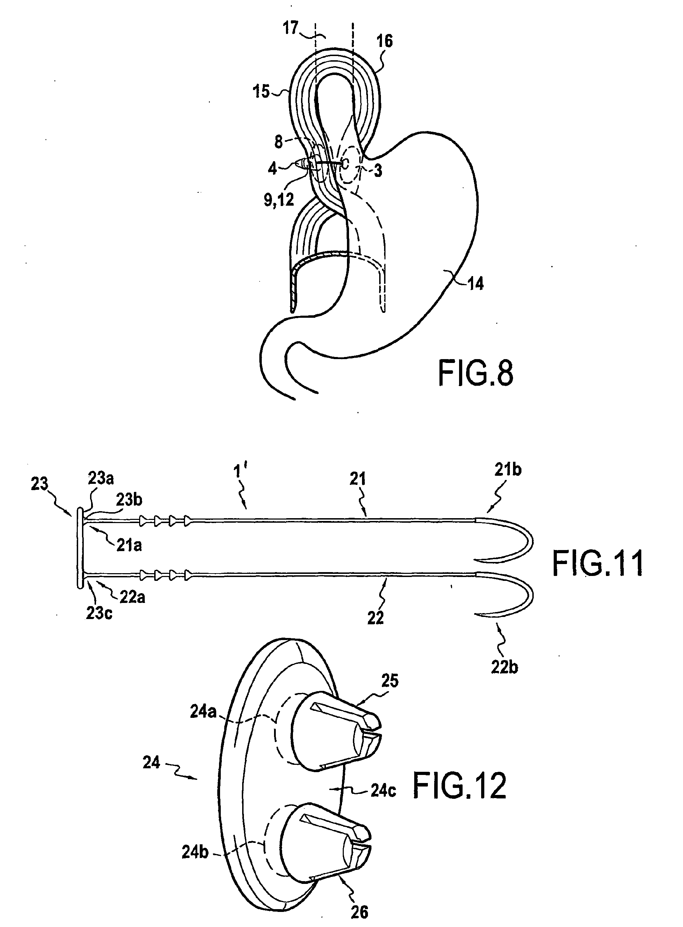

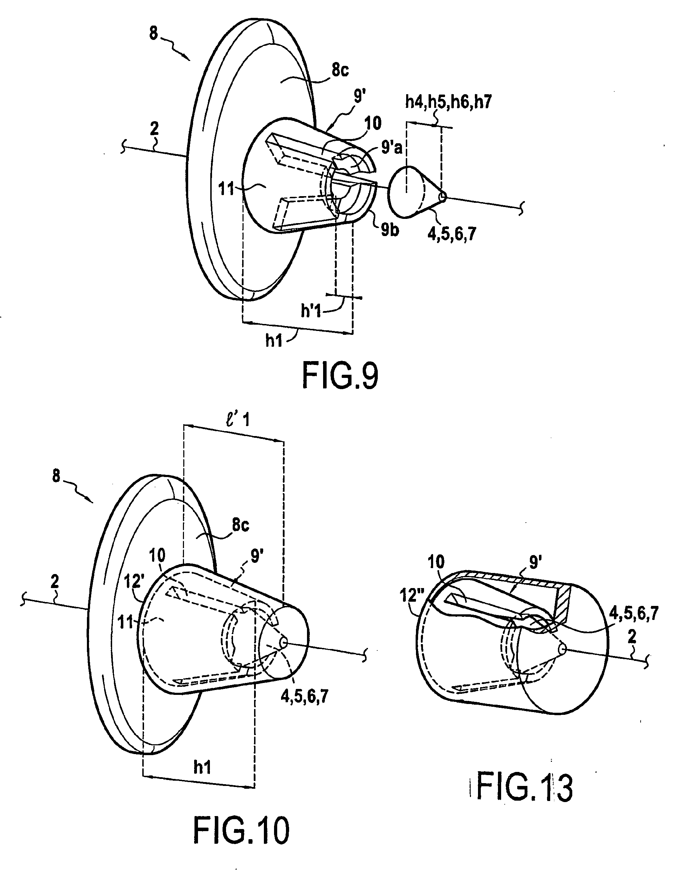

La pièce tronconique 9' représentée aux figures 10 et 11 est une variante de la pièce tronconique 9 décrite ci-dessus. Seuls les éléments de la pièce tronconique 9' qui différent de la pièce 9 sont renumérotés.

La pièce tronconique 9' comporte un épaulement interne 9'a ayant une profondeur h1' dans sa portion d'extrémité 9b formant un logement apte à recevoir une partie de la hauteur h4,h5,h6,h7 de l'une des pièces de blocage 4,5,6,7. La largeur l1' de la bande 12' est de l'ordre de celle de la hauteur h1 de la pièce tronconique 9' majorée de la hauteur h4,h5,h6,h7 moins la profondeur h1' de l'épaulement interne 9a en sorte qu'en fonctionnement (tel que représenté en partie à la figure 10), la bande 12' recouvre à la fois les découpes latérales 10 et la portion de pièce de blocage débouchant de l'épaulement 9a. Ainsi, l'élément longiligne 2 ne risque pas de cisailler les structures anatomiques voisines en passant à travers l'une des découpes, et l'extrémité amincie, voire pointue, de la pièce de blocage ne risque pas de perforer lesdites structures.

De préférence, les première 3 et seconde 8 pièces d'appui, y compris les pièces tronconiques 9,9' ainsi que les pièces de blocage 4,5,6,7 sont moulées, de préférence dans un polymère choisi parmi les polymères suivants : PEEK (polyétherétherkétone), POM (polyoxyméthylène), PET (polyéthylènetéréphtalate), PP (polypropylène), PE (polyéthylène).

Le protecteur 12" représenté à la figure 13 est une variante du protecteur ou bande 12' représenté à la figure 10. Seuls les éléments de la pièce tronconique 9' qui différent de la pièce 9 sont renumérotés à la figure 13. Ce protecteur 12" est moulé en même temps que la seconde pièce d'appui 8 et la première pièce tronconique 9', de préférence dans un polymère choisi parmi les polymères suivants : PEEK (polyétherétherkétone), POM (polyoxyméthylène), PET (polyéthylènetéréphtalate), PP (polypropylène), PE (polyéthylène). Le protecteur 12", étant dans ce cas rigide, se présente sous la forme d'un cylindre s'étendant de la face 8c de la seconde pièce d'appui 8. Le protecteur 12" recouvre les découpes latérales 10 de la pièce tronconique creuse 9' sans entrer en contact direct avec ladite pièce tronconique 9' et donc ses ailettes 11 de sorte de ménager un espace libre, délimité entre ladite pièce tronconique 9' et ledit protecteur 12', dans lequel lesdites ailettes 11 peuvent se déformer radialement lors du passage en force de la pièce de blocage 4,5,6,7.

Le dispositif 1' représenté à la figure 11 diffère du dispositif 1 en ce qu'il comprend deux éléments longilignes 21,22. La première pièce d'appui 23 présente une face intérieure plane 23a suffisante pour être fixée en deux zones de fixations différentes 23b,23c aux extrémités 21a,22a des premier 21 et second 22 éléments. Chaque élément longiligne 21,22 comporte quatre pièces de blocage, de préférence agencées telles que décrites ci-dessus. La seconde pièce d'appui 24, à la figure 12, comporte un premier 24a et un second 24b orifices de passage pour être montée coulissante à la fois respectivement sur le premier 21 et le second 22 éléments longilignes. A l'opposé de la première pièce d'appui 23, les extrémités libres 21b,22b des éléments 21,22 sont rigides et courbes. La seconde pièce d'appui 24 comporte deux pièces tronconiques 25,26 se projetant de sa face extérieure 24c identiques à la pièce tronconique 9 du dispositif 1 et comportant chacune en fonctionnement une bande du type de la bande 12.

En fonctionnement, le praticien passe successivement le premier élément 21 puis le second élément 22 à travers les piliers du diaphragme jusqu'à ce que la première pièce d'appui 23 vienne en butée contre un premier pilier. Puis il enfile la seconde pièce d'appui 24 sur les premier 21 et second 22 éléments longilignes à l'aide des premier 24a et second 24b orifices de passage. Chacune des pièces de blocage disposées sur la longueur des premier 21 et second 22 éléments est forcée à travers les premier 24a et second 24b orifices de passage. De préférence, afin d'obtenir un espace délimité par une dimension sensiblement constante, correspondant à l'écartement entre les première 23 et seconde 24 pièces d'appui, le praticien bloque la seconde pièce d'appui 24 à l'aide de deux pièces de blocage disposées sensiblement à une distance du même ordre de la première d'appui 23.

Le second élément longiligne 22, tout comme le premier élément longiligne 21, ne cisaille pas les piliers et renforce la pression de serrage exercée par le dispositif 1' et notamment les première 23 et seconde 24 pièces d'appui. Le dispositif 1' permet l'emploi de pièces d'appui 23,24 ayant des surfaces d'appui plus importantes que celles des pièces 3,8 du dispositif 1, et de mieux équilibrer la répartition des tensions exercées par les premier 21 et second 22 éléments longilignes entre lesdites pièces 23,24.

De préférence, les pièces tronconiques 25,26 comportent chacune un protecteur se présentant sous la forme d'un tube dans un matériau élastique adhérent équivalent au protecteur 12 du dispositif 1.

Bien sûr, selon la pathologie, il est possible d'implanter un ou plusieurs dispositifs 1 décrits aux figures 1A-1D à 8 et/ou un ou plusieurs dispositifs 1' décrits aux figures 9A et 9B.

## Revendications

1. Dispositif implantable (1,1') pour le rapprochement de structures anatomiques fragiles, notamment de structures musculaires telles que la paroi abdominale , les piliers du diaphragme dans le traitement de la hernie hiatale, le coeur, l'estomac ou l'utérus ou de structures parenchymateuses telles que les reins, le foie et les poumons comprenant au moins un premier élément longiligne (2,21) de rapprochement d'au moins deux desdites structures anatomiques (15,16) comportant sur sa longueur une première pièce d'appui (3), fixe ou fixable dans une position donnée dudit premier élément (2, 21), au moins une pièce de blocage (4,5,6,7) fixée à une distance donnée D (D1,D2,D3,D4) de ladite première pièce d'appui (3) dans ladite position_{;} et une seconde pièce d'appui (8) qui présente un premier orifice (8a,24a) permettant le passage du premier élément longiligne (2,21) et qui est apte à coulisser sur la longueur dudit premier élément longiligne (2,21) en sorte que, lors de l'implantation, ladite pièce de blocage (4,5,6,7) est forcée à travers ladite seconde pièce d'appui (8) et vient en butée contre ladite seconde pièce d'appui (8), lesdites première (3) et seconde (8) pièces d'appui délimitant entre elles un espace ayant au moins une dimension D'inférieure ou égale à D et apte à recevoir et enserrer lesdites structures anatomiques (15,16), **caractérisé en ce que** la seconde pièce d'appui (8,24) comporte une première pièce tronconique creuse (9,9',25) ayant des découpes (10) formant des ailettes latérales (11) et ayant un évidement central (9a) qui prolonge ledit premier orifice de passage (8a, 24a, 25a), le diamètre de sortie de l'évidement (9a) de la première pièce tronconique (9,9',25) étant inférieur à l'encombrement de ladite pièce de blocage (4,5,6,7) en sorte que le passage à force de ladite pièce de blocage (4,5,6,7) à travers la seconde pièce d'appui (8,24) est obtenu grâce à la déformation radiale des ailettes (11), et **en ce que** la première pièce tronconique, est pourvue d'un protecteur (12,12') ayant une largeur (ℓ1, ℓ1') de l'ordre de la hauteur (h1) de ladite pièce tronconique (9,9',25, 26), éventuellement majorée de tout ou partie de la hauteur (h4, h5, h6, h7) de ladite pièce de blocage (4,5,6,7), et apte à être disposé en fonctionnement sur la première pièce tronconique (9,9',25,26), et éventuellement ladite pièce de blocage (4,5,6,7), en sorte de recouvrir au moins lesdites découpes latérales (10).

2. Dispositif (1,1') selon la revendication 1 **caractérisé en ce que** les première (3) et seconde (8) pièces d'appui ont au moins une face intérieure (3a,8b) sensiblement plane, de préférence ladite face intérieure (3a,8b) a une forme générale d'ellipse.

3. Dispositif (1') selon l'une ou l'autre des revendications 1 et 2, **caractérisé en ce qu'**il comprend un second élément longiligne (22) de rapprochement desdites structures anatomiques (15,16), la première pièce d'appui (23) étant fixe ou fixable dans une position donnée dudit second élément (22), et au moins une pièce de blocage fixée sur la longueur dudit second élément à une distance donnée D de ladite première pièce d'appui (23) dans ladite position, la seconde pièce d'appui (24) étant apte à coulisser sur la longueur dudit second élément longiligne (22) en sorte que, lors de l'implantation, ladite pièce de blocage est forcée à travers ladite seconde pièce d'appui (24) et vient en butée contre ladite seconde pièce d'appui (24).

4. Dispositif (1,1') selon l'une des revendications 1 à 3, **caractérisé en ce que** le premier (2,21), et éventuellement le second (22), élément longiligne comporte plusieurs pièces de blocage successives (4,5,6,7), de préférence ayant une hauteur (h4,h5,h6,h7) du même ordre, à des distances prédéterminées D (D1,D2,D3,D4) de ladite première pièce d'appui (3, 23), de préférence la distance D (D1,D2,D3,D4) est comprise dans l'intervalle [10 ; 25] mm.

5. Dispositif (1,1') selon l'une des revendications 1 à 4, **caractérisé en ce que** la seconde pièce d'appui (8,24) présente un second orifice (24b) permettant le passage du second élément longiligne (22).

6. Dispositif (1,1') selon la revendication 5, **caractérisé en ce que** la seconde pièce d'appui (8,24) comporte une seconde pièce tronconique creuse (26), ayant des découpes (10) formant des ailettes latérales (11) et ayant un évidement central (9a) qui prolonge le second orifice de passage (8a, 24a, 25a), et **en ce que** le diamètre de sortie de l'évidement (9a) de ladite seconde pièce tronconique (9,9',25) est inférieur à l'encombrement de la pièce de blocage (4,5,6,7), en sorte que le passage à force de la pièce de blocage (4,5,6,7) à travers la seconde pièce d'appui (8,24) est obtenu grâce à la déformation radiale des ailettes (11).

7. Dispositif (1) selon la revendication 6, **caractérisé en ce que** la première (9'), et éventuellement la seconde, pièce tronconique creuse comporte un épaulement interne (9'a) dans sa portion d'extrémité (9b) formant un logement apte à recevoir tout ou partie de la hauteur (h4,h5,h6,h7) de l'une des pièces de blocage (4,5,6,7).

8. Dispositif (1, 1') selon l'une ou l'autre des revendications 6 et 7, **caractérisé en ce que** ladite seconde (26) pièce tronconique est pourvue d'un protecteur (12,12') ayant une largeur (f1, ℓ1') de l'ordre de la hauteur (h1) de ladite seconde pièce tronconique (9,9',25, 26), éventuellement majorée de tout ou partie de la hauteur (h4, h5, h6, h7) de l'une des pièces de blocage (4,5,6,7), et apte à être disposée en fonctionnement sur ladite seconde pièce tronconique (9,9',25,26), et éventuellement ladite pièce de blocage (4,5,6,7), en sorte de recouvrir au moins lesdites découpes latérales (10).

9. Dispositif selon l'une des revendications 1 à 8, **caractérisé en ce que** le protecteur de la première pièce tronconique creuse, et éventuellement de la seconde pièce tronconique creuse, consiste dans une bande dans un matériau élastique, de préférence à base de silicone.

10. Dispositif (1,1') selon l'une des revendications 1 à 9, **caractérisé en ce que** le premier élément longiligne (2,21), et éventuellement le second élément longiligne (22), est un monofilament, une tresse ou un câble rigide de faible diamètre, de préférence de l'ordre de 1,7 mm.

11. Dispositif (1,1') selon l'une des revendications 1 à 10, **caractérisé en ce que** ledit premier (2,21), et éventuellement ledit second (22), élément longiligne présente une extrémité rigide et courbe (2b,21b,22b) permettant son introduction dans lesdites structures anatomiques, notamment à l'opposé de ladite première pièce d'appui (3,23).

12. Dispositif (1,1') selon l'une des revendications 1 à 11, **caractérisé en ce que** la ou les pièces de blocage (4,5,6,7) ont une forme générale tronconique.

13. Dispositif (1,1') selon l'une des revendications 1 à 12, **caractérisé en ce que** les première (3,23) et seconde (8,24) pièces d'appui, la ou les pièces de blocage (4,5,6,7), la première pièce tronconique creuse (9,9',25), éventuellement la seconde pièce tronconique creuse (26), le protecteur de la première pièce tronconique creuse (12"), et éventuellement le protecteur de la seconde pièce tronconique creuse, sont moulés, de préférence dans un polymère choisi parmi les polymères suivants : PEEK (polyétherétherkétone), POM (polyoxyméthylène), PET (polyéthylènetéréphtalate), PP (polypropylène), PE (Polyéthylène).

14. Ensemble pour le rapprochement de structures anatomiques fragiles, notamment de structures musculaires telles que la paroi abdominale, les piliers du diaphragme dans le traitement de la hernie hiatale, le coeur, l'estomac ou l'utérus, ou de structures parenchymateuses telles que les reins, le foie et les poumons comportant un dispositif implantable (1,1') selon l'une des revendications 1 à 13 et un kit d'implantation comprenant notamment un organe de traction (19) et un poussoir (20).

## Claims

1. An implantable device (1, 1') for bringing together fragile anatomical structures, in particular muscular structures such as the abdominal wall, the pillars of the diaphragm in treating a hiatus hernia, the heart, the stomach, or the uterus, or for bringing together parenchymatous structures such as the kidneys, the liver, and the lungs, said implantable device comprising at least a first filamentary element (2, 21) for bringing together at least two of said anatomical structures (15, 16), which element, along its length, has a first bearing piece (3) that is stationary or fastenable in a given position along said first element (2, 21), at least one locking piece (4, 5, 6, 7) fastened at a given distance D (D1, D2, D3, D4) from said first bearing piece (3) in said position, and a second bearing piece (8) that has a first through orifice (8a, 24a) allowing the first filamentary element (2, 21) to pass through it, and that is suitable for sliding along said first filamentary element (2, 21) so that, during the implantation, said locking piece (4, 5, 6, 7) is forced through said second bearing piece (8) and comes into abutment against said second bearing piece (8), said first and second bearing pieces (3, 8) defining between them a space that has at least one dimension D' less than or equal to D, and that is suitable for receiving and clamping said anatomical structures (15, 16), said implantable device being **characterized in that** the second bearing piece (8, 24) has a first hollow frustoconical piece (9, 9', 25) having cutouts (10) forming side fins (11) and having a central cavity (9a) that extends said first through orifice (8a, 24a, 25a), the outlet diameter of the cavity (9a) of the first frustoconical piece (9, 9', 25) being smaller than the overall size of said locking piece (4, 5, 6, 7) so that said locking piece (4, 5, 6, 7) being forced through the second bearing piece (8, 24) is obtained by means of the fins (11) deforming radially, and **in that** the first frustoconical piece is provided with a guard (12, 12') having a width (ℓ1, ℓ1') approximately equal to the height (h1) of said frustoconical piece (9, 9', 25, 26), optionally plus all or some of the height (h4, h5, h6, h7) of said locking piece (4, 5, 6, 7), and suitable for being disposed, in operation, over the first frustoconical piece (9, 9', 25, 26) and optionally over said locking piece (4, 5, 6, 7), so as to cover at least said side cutouts (10).

2. A device (1, 1') according to claim 1, **characterized in that** each of the first and second bearing pieces (3, 8) has at least one inside face (3a, 8b) that is substantially plane, and preferably said inside face (3a, 8b) is elliptical in general shape.

3. A device (1') according to claim 1 or claim 2, **characterized in that** it further comprises a second filamentary element (22) for bringing together said anatomical structures (15, 16), the first bearing piece (23) being stationary or fastenable in a given position along said second element (22), and at least one locking piece fastened along the length of said second element at a given distance D from said first bearing piece (23) in said position, the second bearing piece (24) being suitable for sliding along said second filamentary element (22) so that, during the implantation, said locking piece is forced through said second bearing piece (24) and comes into abutment against said second bearing piece (24).

4. A device (1, 1') according to any one of claims 1 to 3, **characterized in that** the first and optionally the second filamentary element (2, 21, 22) has successive locking pieces (4, 5, 6, 7) that preferably have heights (h4, h5, h6, h7) of the same order of magnitude, and that are situated at predetermined distances D (D1, D2, D3, D4) from said bearing piece (3, 23), and preferably the distance D (D1, D2, D3, D4) lies in the range 10 mm to 25 mm.

5. A device (1, 1') according to any one of claims 1 to 4, **characterized in that** the second bearing piece (8, 24) has a second through orifice (24b) making it possible for the second filamentary element (22) to pass through it.

6. A device (1, 1') according to claim 5, **characterized in that** the second bearing piece (8, 24) has a second hollow frustoconical piece (26) having cutouts (10) forming side fins (11) and having a central cavity (9a) that extends the second through orifice (8a, 24a, 25a), and **in that** the outlet diameter of the cavity (9a) of said second frustoconical piece (9, 9', 25) is less than the overall size of the locking piece (4, 5, 6, 7), so that the locking piece (4, 5, 6, 7) being forced through the second bearing piece (8, 24) is obtained by means of the fins (11) deforming radially.

7. A device (1) according to claim 6, **characterized in that** the first and optionally the second hollow frustoconical piece (9') has an internal shoulder (9'a) in its end portion (9b), which internal shoulder forms a recess suitable for receiving all or some of the height (h4, h5, h6, h7) of one of the locking pieces (4, 5, 6, 7).

8. A device (1, 1') according to claim 6 or claim 7, **characterized in that** said second frustoconical piece (26) is provided with a guard (12, 12') having a width (ℓ1, ℓ1') approximately equal to the height (h1) of said second frustoconical piece (9, 9', 25, 26) optionally plus all or some of the height (h4, h5, h6, h7) of one of the locking pieces (4, 5, 6, 7), and suitable for being disposed, in operation, over said second frustoconical piece (9, 9', 25, 26), and optionally over said locking piece (4, 5, 6, 7), so as to cover at least said side cutouts (10).

9. A device according to any one of claims 1 to 8, **characterized in that** the guard of the first and optionally the guard of the second hollow frustoconical piece consists of a band of an elastic material, preferably based on silicone.

10. A device (1, 1') according to any one of claims 1 to 9, **characterized in that** the first and optionally second filamentary element (2, 21, 22) is a monofilament, a braid, or a rigid cable of small diameter, preferably approximately equal to 1.7 mm.

11. A device (1, 1') according to any one of claims 1 to 10, **characterized in that** said first and optionally second filamentary element (2, 21, 22) has a rigid and curved end (2b, 21b, 22b) enabling it to be inserted into said anatomical structures, in particular opposite from said first bearing piece (3, 23).

12. A device (1, 1') according to any one of claims 1 to 11, **characterized in that** the locking pieces (4, 5, 6, 7) are frustoconical in general shape.

13. A device (1, 1') according to any one of claims 1 to 12, **characterized in that** the first and second bearing pieces (3, 23, 8, 24), the locking pieces (4, 5, 6, 7), the first hollow frustoconical piece (9, 9', 25), optionally the second hollow frustoconical piece (26), the guard (12") of the first hollow frustoconical piece, and optionally the guard of the second hollow frustoconical piece are molded, preferably of a polymer chosen from the following polymers: polyetheretherketone (PEEK), polyoxymethylene (POM), polyethyleneterephtalate (PET), polypropylene (PP), and polyethylene (PE).

14. A set for bringing together fragile anatomical structures, in particular muscular structures such as the abdominal wall, the pillars of the diaphragm in treating a hiatus hernia, the heart, the stomach, or the uterus, or for bringing together parenchymatous structures such as the kidneys, the liver, and the lungs, said set comprising an implantable device (1, 1') according to any one of claims 1 to 13, and an implantation kit including, in particular, a traction member (19) and a pusher (20).

## Patentansprüche

1. Implantierbare Vorrichtung (1,1') für die Annäherung fragiler anatomischer Strukturen, insbesondere von Muskelstrukturen, wie der Bauchwandung, der Zwerchfellpfeiler bei der Behandlung des Zwerchfellbruchs, des Herzens, des Magens oder der Gebärmutter, oder von parenchymatösen Strukturen, wie den Nieren, der Leber und den Lungen, umfassend wenigstens ein erstes langgestrecktes Element (2, 21) zur Annäherung von wenigstens zwei der anatomischen Strukturen (15, 16), das über seine Länge ein erstes Anlageteil (3), welches in einer gegebenen Position des ersten Elements (2, 21) fest oder fixierbar ist, wenigstens ein Blockierungsteil (4, 5, 6, 7), welches in einem gegebenen Abstand D (D1, D2, D3, D4) von dem ersten Anlageteil (3) in der genannten Position festgelegt ist, sowie ein zweites Anlageteil (8) umfaßt, das eine den Durchgang des ersten langgestreckten Elements (2, 21) ermöglichende erste Öffnung (8a, 24a) aufweist und das über die Länge des ersten langgestreckten Elements (2, 21) derart verschieblich ist, daß während des Implantierens das Blockierungsteil (4, 5, 6, 7) durch das zweite Anlageteil (8) gedrückt wird und an dem zweiten Anlageteil (8) in Anschlag gelangt, wobei das erste Anlageteil (3) und das zweite Anlageteil (8) zwischen sich einen Raum begrenzen, der wenigstens eine Abmessung D' aufweist, die kleiner als oder gleich D ist, und geeignet ist, die anatomischen Strukturen (15, 16) aufzunehmen und festzuklemmen, **dadurch gekennzeichnet, daß** das zweite Anlageteil (8, 24) ein erstes kegelstumpfförmiges Hohlteil (9, 9', 25) umfaßt, das Seitenflügel (11) bildende Ausschnitte (10) sowie eine mittlere Aussparung (9a) aufweist, die die erste Durchgangsöffnung (8a, 24a, 25a) fortsetzt, wobei der Austrittsdurchmesser der Aussparung (9a) des ersten kegelstumpfförmigen Teils (9, 9', 25) kleiner als der Platzbedarf des Blockierungsteils (4, 5, 6, 7) ist, so daß das Hindurchdrücken des Blockierungsteils (4, 5, 6, 7) durch das zweite Anlageteil (8, 24) dank der radialen Verformung der Flügel (11) erreicht wird, und daß das erste kegelstumpfförmige Teil mit einer Schutzabdeckung (12, 12') versehen ist, die eine Breite (l1, l1') in der Größenordnung der Höhe (h1) des kegelstumpfförmigen Teils (9, 9', 25, 26) aufweist, eventuell erhöht um die gesamte Höhe (h4, h5, h6, h7) des Blockierungsteils (4, 5, 6, 7) oder einen Teil dieser Höhe, und die geeignet ist, im Betrieb auf dem ersten kegelstumpfförmigen Teil (9, 9', 25, 26) und eventuell dem Blockierungsteil (4, 5, 6, 7) angeordnet zu werden, um wenigstens die seitlichen Ausschnitte (10) zu bedecken.

2. Vorrichtung (1, 1') nach Anspruch 1, **dadurch gekennzeichnet, daß** das erste Anlageteil (3) und das zweite Anlageteil (8) wenigstens eine im wesentlichen ebene Innenseite (3a, 8b) aufweisen, vorzugsweise ist die Innenseite (3a, 8b) allgemein ellipsenförmig ausgebildet.

3. Vorrichtung (1, 1') nach dem einen oder anderen der Ansprüche 1 und 2, **dadurch gekennzeichnet, daß** sie ein zweites langgestrecktes Element (22) zur Annäherung der anatomischen Strukturen (15, 16), wobei das erste Anlageteil (23) in einer gegebenen Position des zweiten Elements (22) fest oder fixierbar ist, sowie wenigstens ein Blockierungsteil umfaßt, welches über die Länge des zweiten Elements in einem gegebenen Abstand D von dem ersten Anlageteil (23) in der genannten Position festgelegt ist, wobei das zweite Anlageteil (24) über die Länge des zweiten langgestreckten Elements (22) derart verschieblich ist, daß während des Implantierens das Blockierungsteil durch das zweite Anlageteil (24) gedrückt wird und an dem zweiten Anlageteil (24) in Anschlag gelangt

4. Vorrichtung (1, 1') nach einem der Ansprüche 1 bis 3, **dadurch gekennzeichnet, daß** das erste (2, 21) und eventuell das zweite (22) langgestreckte Element mehrere aufeinanderfolgende Blockierungsteile (4, 5, 6, 7), vorzugsweise mit einer Höhe (h4, h5, h6, h7) der gleichen Größenordnung, in vorbestimmten Abständen D (D1, D2, D3, D4) vom ersten Anlageteil (3, 23) umfaßt, vorzugsweise liegt der Abstand D (D1, D2, D3, D4) im Bereich [10; 25] mm.

5. Vorrichtung (1, 1') nach einem der Ansprüche 1 bis 4, **dadurch gekennzeichnet, daß** das zweite Anlageteil (8, 24) eine zweite Öffnung (24b) aufweist, die den Durchgang des zweiten langgestreckten Elements (22) ermöglicht.

6. Vorrichtung (1, 1') nach Anspruch 5, **dadurch gekennzeichnet, daß** das zweite Anlageteil (8, 24) ein zweites kegelstumpfförmiges Hohlteil (26) umfaßt, das Seitenflügel (11) bildende Ausschnitte (10) sowie eine mittlere Aussparung (9a) aufweist, die die zweite Durchgangsöffnung (8a, 24a, 25a) fortsetzt, und daß der Austrittsdurchmesser der Aussparung (9a) des zweiten kegelstumpfförmigen Teils (9, 9', 25) kleiner als der Platzbedarf des Blockierungsteils (4, 5, 6, 7) ist, so daß das Hindurchdrücken des Blockierungsteils (4, 5, 6, 7) durch das zweite Anlageteil (8, 24) dank der radialen Verformung der Flügel (11) erreicht wird.

7. Vorrichtung (1) nach Anspruch 6, **dadurch gekennzeichnet, daß** das erste (9') und eventuell das zweite kegelstumpfförmige Hohlteil eine Innenschulter (9'a) in seinem Endabschnitt (9b) aufweist, der eine Aufnahme bildet, welche geeignet ist, die gesamte Höhe (h4, h5, h6, h7) von einem der Blockierungsteile (4, 5, 6, 7) oder einen Teil dieser Höhe aufzunehmen.

8. Vorrichtung (1,1') nach dem einen oder anderen der Ansprüche 6 und 7, **dadurch gekennzeichnet, daß** das zweite kegelstumpfförmige Teil (26) mit einer Schutzabdeckung (12, 12') versehen ist, die eine Breite (11, 11') in der Größenordnung der Höhe (h1) des zweiten kegelstumpfförmigen Teils (9, 9', 25, 26) aufweist, eventuell erhöht um die gesamte Höhe (h4, h5, h6, h7) von einem der Blockierungsteile (4, 5, 6, 7) oder einen Teil dieser Höhe, und die geeignet ist, im Betrieb auf dem zweiten kegelstumpfförmigen Teil (9, 9`, 25, 26) und eventuell dem Blockierungsteil (4, 5, 6, 7) angeordnet zu werden, um wenigstens die seitlichen Ausschnitte (10) zu bedecken.

9. Vorrichtung nach einem der Ansprüche 1 bis 8, **dadurch gekennzeichnet, daß** die Schutzabdeckung des ersten kegelstumpfförmigen Hohlteils und eventuell des zweiten kegelstumpfförmigen Hohlteils aus einem Band aus einem elastischen Material, vorzugsweise auf Silikonbasis besteht.

10. Vorrichtung (1, 1') nach einem der Ansprüche 1 bis 9, **dadurch gekennzeichnet, daß** das erste langgestreckte Element (2, 21) und eventuell das zweite langgestreckte Element (22) ein Monofilament, eine Schnur oder ein starres Seil mit geringem Durchmesser, vorzugsweise in der Größenordnung von 1,7 mm ist.

11. Vorrichtung (1,1') nach einem der Ansprüche 1 bis 10, **dadurch gekennzeichnet, daß** das erste (2, 21) und eventuell das zweite (22) langgestreckte Element ein starres und gebogenes Ende (2b, 21 b, 22b), insbesondere gegenüber dem ersten Anlageteil (3, 23), aufweist, das sein Einführen in die anatomischen Strukturen ermöglicht.

12. Vorrichtung (1,1') nach einem der Ansprüche 1 bis 11, **dadurch gekennzeichnet, daß** das oder die Blockierungsteile (4, 5, 6, 7) allgemein kegelstumpfförmig ausgebildet sind.

13. Vorrichtung (1, 1') nach einem der Ansprüche 1 bis 12, **dadurch gekennzeichnet, daß** das erste Anlageteil (3, 23) und das zweite Anlageteil (8, 24), das oder die Blockierungsteile (4, 5, 6, 7), das erste kegelstumpfförmige Hohlteil (9, 9', 25), eventuell das zweite kegelstumpfförmige Hohlteil (26), die Schutzabdeckung des ersten kegelstumpfförmigen Hohlteils (12") und eventuell die Schutzabdeckung des zweiten kegelstumpfförmigen Hohlteils geformt sind, vorzugsweise aus einem Polymer, das aus den folgenden Polymeren ausgewählt ist, nämlich PEEK (Polyetheretherketon), POM (Polyoxymethylen), PET (Polyethylenterephtalat), PP (Polypropylen), PE (Polyethylen).

14. Anordnung für die Annäherung fragiler anatomischer Strukturen, insbesondere von Muskelstrukturen, wie der Bauchwandung, der Zwerchfellpfeiler bei der Behandlung des Zwerchfellbruchs, des Herzens, des Magens oder der Gebärmutter, oder von parenchymatösen Strukturen, wie den Nieren, der Leber und den Lungen, umfassend eine implantierbare Vorrichtung (1, 1') nach einem der Ansprüche 1 bis 13 und ein Implantationsset, das insbesondere ein Zugorgan (19) und eine Druckvorrichtung (20) umfaßt.
